# EUROPÄISCHE PATENTSCHRIFT

(11) **EP 2 340 432 B1**
(45) Veröffentlichungstag und Bekanntmachung des Hinweises auf die Patenterteilung: **08.11.2017**
(21) Anmeldenummer: 09783492.3
(22) Anmeldetag: 28.09.2009
(51) Int. Cl.: G01N 21/31, G01N 33/18, G01N 27/28, G01N 27/403, B01L 3/00

(54) **VERFAHREN ZUR BESTIMMUNG EINES ANALYTS IN EINER WASSERPROBE MIT HILFE EINER MOBILEN WASSER-ANALYSEANORDNUNG**
METHOD FOR DETERMINING AN ANALYTE IN A WATER SAMPLE BY MEANS OF A MOBILE WATER ANALYSIS ARRANGEMENT
PROCÉDÉ D'ANALYSE D'UN ANALYTE DANS UN ÉCHANTILLON D'EAU À L'AIDE D'UN DISPOSITIF D'ANALYSE D'EAU MOBILE

(30) Priorität: 06.10.2008 DE 102008050092
(43) Veröffentlichungstag der Anmeldung: 06.07.2011
(73) Patentinhaber: Hach Lange GmbH, 14163 Berlin (DE)
(72) Erfinder: LUNDGREEN, Ulrich, 33330 Gütersloh (DE); FARJAM, Aria, 40223 Düsseldorf (DE); UTHEMANN, Rolf, 51373 Leverkusen (DE); MITREITER, Andreas, 14532 Kleinmachnow (DE); HÜNIG, Isabel, 40547 Düsseldorf (DE); LENHARD, Markus, 41751 Viersen (DE); FRÖMEL, Rainer, 53842 Troisdorf (DE); KUMPCH, Hans-Joachim, 10999 Berlin (DE)
(74) Vertreter: Patentanwälte ter Smitten Eberlein-Van Hoof Rütten Partnerschaftsgesellschaft mbB
(86) Internationale Anmeldenummer: PCT/EP2009/062535
(87) Internationale Veröffentlichungsnummer: WO 2010/040657

(56) Entgegenhaltungen:
- EP-A2- 0 821 231
- WO-A2-02/074043
- DE-A1- 10 126 054
- US-A- 5 731 212
- US-A- 5 821 405
- US-A1- 2004 154 933
- US-A1- 2007 144 277

## Beschreibung

Die Erfindung bezieht sich auf ein Verfahren zur Bestimmung eines Analyts in einer Wasserprobe mit Hilfe einer mobilen Wasser-Analyseanordnung. Unter "mobil" ist vorliegend eine Analyseanordnung zu verstehen, die nicht stationär verbaut ist, wie dies beispielsweise bei quasi-kontinuierlich messenden Prozess-Analysegeräten der Fall ist.

Im Bereich der mobilen Wasseranalytik stellen gegenwärtig u.a. sogenannte fotometrische Küvettentests den Stand der Technik dar, wie er beispielsweise in DE 41 09 118 A1 beschrieben ist. Die Durchführung des Küvettentests erfolgt praktisch rein manuell. Zunächst wird mit einer Pipette eine Wasserprobe aufgenommen. Die auf diese Weise gewonnene Wasserprobe wird in eine Küvette gegeben, die zur Ermittlung der Eigenabsorption der Wasserprobe, dem so genannten Probenblindwert, in ein Fotometer eingesetzt und fotometriert wird. Anschließend wird der Wasserprobe in der Küvette ein Reagenz zugefügt. Die Küvette wird verschlossen und zum Vermischen der Wasserprobe mit dem Reagenz geschüttelt. Schließlich wird die Küvette in ein Fotometer eingesetzt und fotometriert.

Die Durchführung des manuellen Küvettentests ist umständlich und äußerst fehleranfällig. Das verwendete Reagenz kann umwelt- oder gesundheitsschädlich sein. Gegebenenfalls muss daher die Küvette nach Durchführung der Analyse entsprechend entsorgt werden. Wegen der umständlichen Handhabung können die Küvettentests praktisch nur im Labor durchgeführt werden.

Aus US 2007/0144277 ist eine modular aufgebaute Analyseanordnung bekannt, die aus einem Basisgerät und einer austauschbaren Einweg-Kartusche besteht. Die Handhabung dieses Analysegerätes ist gegenüber der Handhabung der Küvettentests vereinfacht.

In nicht-mobilen Analyseanordnungen, wie sie beispielsweise aus US 5,731,212 und WO 02/074043 A2 bekannt sind, können derartige Wasserproben-Analysen einschließlich der Ermittlung der Eigenabsorption der Wasserprobe automatisch durchgeführt werden.

Aufgabe der Erfindung ist es, ein Verfahren zur Bestimmung eines Analyts in einer Wasserprobe einschließlich der Ermittlung der Eigenabsorption der Wasserprobe mit einer mobilen Wasser-Analyseanordnung mit vereinfachter Handhabung zu schaffen.

Diese Aufgabe wird erfindungsgemäß durch ein Verfahren gemäß Patentanspruch 1 geschaffen.

Das erfindungsgemäße Verfahren zur Bestimmung eines Analyts in einer Wasserprobe bezieht sich auf eine mobile Wasser-Analyseanordnung mit einer mobilen Basiseinheit und einem austauschbaren Einmal-Testelement, das für die Bestimmung des Analyts in die Basiseinheit eingesteckt wird beziehungsweise in diese einsteckbar ist.

Das Testelement ist ein komplexes Bauteil, das eine Probenleitung mit einer Proben-Einlassöffnung an dem distalen Ende des Testelementes aufweist. Im Verlauf der Probenleitung ist ein Messabschnitt vorgesehen, der eine Messstrecke für ein Messgerät darstellt, das in der Basiseinheit vorgesehen ist. Alle übrigen Bauteile des Messgerätes sind nicht in dem Testelement, sondern in der Basiseinheit angeordnet. Die Basiseinheit weist ferner eine Testelement-Aufnahme zum Halten des eingesteckten Testelementes auf.

Im weiteren Verlauf der Probenleitung, also von der Einlassöffnung aus gesehen hinter dem Messabschnitt, ist ein erster Reagenzabschnitt vorgesehen, in dem ein Reagenz, beispielsweise ein Hauptreagenz oder ein Hilfsreagenz gelagert ist. Ein Hauptreagenz reagiert mit einem zu bestimmenden Analyt der Wasserprobe farbverändernd, bzw. macht es für eine elektrochemische Analyse zugänglich. Ein Hilfsreagenz reagiert ebenfalls mit der Wasserprobe, jedoch nicht unmittelbar mit dem Analyten zu dem Zwecke einer quantitativen Bestimmung des Analyts in der Wasserprobe, sondern zu anderen Zwecken.

Erfindungsgemäß ist zunächst das Einstecken des Testelementes in die Testelement-Aufnahme der Basiseinheit vorgesehen. Dies kann manuell oder automatisch erfolgen. Daraufhin wird die Einlassöffnung des Testelementes manuell oder automatisch in das zu untersuchende Wasser eingetaucht und eine definierte Menge beziehungsweise ein definiertes Volumen einer Wasserprobe durch Vorwärtsfördern gewonnen, die von der Einlassöffnung bis zu dem Messabschnitt gefördert wird.

Die Gewinnung der definierten Menge einer Wasserprobe kann automatisch oder teilautomatisch ablaufen: Durch das Einstecken des Testelementes in die Testelement-Aufnahme der Basiseinheit wird die Basiseinheit für eine Analyt- Bestimmung aktiviert. Anschließend wird die Einlassöffnung manuell oder automatisch in das zu untersuchende Wasser eingetaucht. Nach dem Eintauchen der Einlassöffnung in das Wasser, das beispielsweise durch Detektion eines kleinen Druckstoßes in der Probenleitung festgestellt werden kann, wird der Pumpenaktuator eingeschaltet. Das Einschalten des Pumpenaktuators kann alternativ auch manuell ausgelöst werden. Durch das Einschalten des Pumpenaktuators wird die Wasserprobe durch die Einlassöffnung der Probenleitung aktiv in Richtung Messabschnitt gepumpt.

Es wird eine definierte Menge der Wasserprobe in Form einer Probensäule angesaugt und isoliert, die an beiden Längsenden durch Luft begrenzt ist. Durch die Beschränkung auf eine Probensäule definierten Volumens wird ein definiertes Verhältnis zwischen der Wasserprobe und einem Hauptreagenz sichergestellt.

Die Begrenzung der Wasserproben-Menge auf eine definierte Menge kann beispielsweise dadurch erfolgen, dass nach dem Ansaugen der definierten Wasserproben-Menge der Pumpenaktuator anhält und ein Probennahme-Endsignal ausgibt, das den Bediener auffordert, die Einlassöffnung aus dem zu untersuchenden Wasser wieder heraus zu nehmen. Alternativ kann die Isolation der definierten Wasserproben-Menge auch automatisch über ein entsprechendes Ventil erfolgen, das nach dem Ansaugen der definierten Wasserproben-Säule hinter der Wasserprobe Luft in die Probenleitung einleitet.

Unter Vorwärtsfördern ist vorliegend stets ein Fördern in der Probenleitung weg von der Einlassöffnung zu verstehen. Das Vorwärtsfördern kann auf jedwede bekannte Weise erfolgen, also beispielsweise durch eine an die Probenleitung angeschlossene basiseinheitsseitige Pumpe, oder durch eine ein relativ großes Pumpenvolumen einschließende Pumpenmembran auf dem Einmal-Testelement, die durch einen entsprechenden basiseinheitsseitigen Pumpenaktuator, beispielsweise einen angetriebenen Pumpenstößel, betätigt wird.

In dem Messabschnitt wird mit Hilfe des Messgerätes eine erste Analyse der Wasserprobe durchgeführt, indem ein Probenblindwert bestimmt wird.

Das Messgerät kann ein elektrochemisches Messgerät sein, das eine elektrische Größe der Wasserprobe bestimmt, kann jedoch alternativ oder ergänzend ein Fotometer mit einer Lichtquelle und einem Lichtempfänger sein.

Sobald die erste Analyse abgeschlossen ist, wird die Wasserprobe aus dem Messabschnitt vorwärts in den ersten Reagenzabschnitt gefördert. In dem ersten Reagenzabschnitt stößt die Wasserprobe auf das erste Reagenz und vermischt sich dort mit dem ersten Reagenz. Das erste Reagenz kann ein Hauptreagenz z. B. zur farbverändernden Reaktion mit dem zu bestimmenden Analyt der Wasserprobe sein. Das erste Reagenz kann jedoch alternativ auch ein Hilfsreagenz sein.

Das erste Reagenz ist bevorzugt ein Hauptreagenz, und geht bevorzugt mit dem nachzuweisenden Analyt in der Wasserprobe eine Reaktion ein, die die optischen oder elektrochemischen Eigenschaften der Wasserprobe verändert. Bezogen auf das Beispiel eines als Fotometer ausgebildeten Messgerätes wird insbesondere das Absorptionsspektrum der Wasserprobe auf bestimmten Spektrallinien beziehungsweise in bestimmten Spektralbereichen verändert. Das erste Reagenz kann jedoch auch ein Hilfsreagenz, das kein Nachweis-Reagenz ist; sein.

Die auf diese Weise aufbereitete und durch Vermischen homogenisierte Wasserprobe, was beispielsweise durch mehrfaches Hin- und Herpumpen vorgenommen wird, gelangt schließlich von dem ersten Reagenzabschnitt durch Rückwärtsfördern zurück in den Messabschnitt, in dem sie durch das Messgerät der Basiseinheit elektrisch und/oder optisch einer zweiten Analyse unterzogen wird. Das Ergebnis dieser zweiten Analyse ist beispielsweise ein Brutto-Messwert. Schließlich wird beispielsweise der Blindprobenwert der ersten Analyse von dem Brutto-Messwert der zweiten Analyse subtrahiert, um auf diese Weise den Netto-Messwert für das Analyt zu erhalten. Der Netto-Messwert wird gegebenenfalls angezeigt und/oder abgespeichert. Sobald der Netto-Messwert vorliegt, kann das Einmal-Testelement manuell oder automatisch entnommen beziehungsweise entfernt werden.

Alle für die Qualität des Messergebnisses relevanten Arbeitsschritte der Wasseranalyse, insbesondere die Analyse, beispielsweise die Bestimmung eines Probenblindwertes, die Dosierung des ersten Reagenzes, die Vermischung des ersten Reagenzes mit der Wasserprobe, das Abwarten der erforderlichen Reaktionszeit etc. erfolgen halbautomatisch beziehungsweise automatisch und verschlossen. Damit sind aus einer fehlerhaften Handhabung resultierende Fehler und Gefährdungen nahezu ausgeschlossen.

Gegenstand des Patentanspruches 1 ist im wesentlichen ein Verfahren für ein Einmal-Testelement mit einer Probenleitung und eine Messabschnitt, bei dem nach einer ersten Analyse der Wasserprobe in dem Messabschnitt die Wasserprobe weiter vorwärts in einen ersten Reagenzabschnitt gepumpt, dort mit dem Reagenz vermischt, zurück in den Messabschnitt gepumpt und dort erneut analysiert wird. Hierbei gelangt die Wasserprobe vor der ersten Analyse zur Ermittlung eines Probenblindwertes reagenzfrei zu der ersten Analyse in den Messabschnitt. Durch die Anordnung eines Reagenzes in einem ersten Reagenzabschnitt hinter dem Messabschnitt werden für die Durchführung einer beziehungsweise mehrerer Analysen und die Verbesserung weiterer Eigenschaften des Einmal-Testelements eine Vielzahl von Möglichkeiten geschaffen.

Gemäß einer bevorzugten Ausgestaltung des erfindungsgemäßen Verfahrens ist in der Probenleitung hinter dem ersten Reagenzabschnitt ein zweiter Reagenzabschnitt, bevorzugt mit einer Hilfssubstanz, vorgesehen. An die Wasserproben-Analyse schließen sich folgende Verfahrensschritte an:
Vorwärtsfördern in der analysierten Wasserprobe von dem Messabschnitt in den zweiten Reagenzabschnitt.

In dem zweiten Reagenzabschnitt reagiert die Wasserprobe mit dem zweiten Reagenz, also bevorzugt mit einer Hilfssubstanz. Durch das Vorsehen einer zweiten Reagenz, vom Messabschnitt aus gesehen, hinter und ausserhalb des ersten Reagenzes beziehungsweise Reagenzabschnittes, kann eine Vielzahl verschiedener zusätzlicher Funktionen realisiert werden. Beispielsweise kann das zweite Reagenz eine Hilfssubstanz in Form eines Geliermittels und/oder ein Färbemittels sein, das die Wasserprobe im Anschluss an die Analyse verdickt und/oder einfärbt. Hierdurch wird ein Auslaufen der Wasserprobe aus der Probenleitung verhindert beziehungsweise, durch die Einfärbung der Wasserprobe, dem Benutzer und/oder dem Basisgerät, das die Veränderung der optischen Eigenschaft feststellen kann, signalisiert, dass das Einmal-Testelement bereits benutzt wurde.

Besonders bevorzugt wird die Wasserprobe aus dem zweiten Reagenzabschnitt rückwärts zurück in den Messabschnitt gefördert, wo die Wasserprobe mit Hilfe des Messgerätes ein drittes Mal analysiert wird. Hinter dem zweiten Reagenzabschnitt kann ein dritter Reagenzabschnitt, beispielsweise mit einer zweiten Hilfssubstanz, vorgesehen seien, wobei sich an das dritte Analysieren der Wasserprobe anschließt:
Vorwärtsfördern der Wasserprobe in den dritten Reagenzabschnitt, Rückwärtsfördern der Wasserprobe aus dem dritten Reagenzabschnitt in den Messabschnitt, und
viertes Analysieren der Wasserprobe mit Hilfe des Messgerätes.

Die beiden Hilfssubstanzen in den beiden Hilfssubstanzabschnitten, also in dem zweiten und dritten Reagenzabschnitt, sind bevorzugt Analyt-Standards verschiedener Menge beziehungsweise Konzentration. Auf diese Weise lässt sich mit dem beschriebenen Verfahren ein Standard-Additionsverfahren durchführen, das für jede Messsequenz eine Kalibrierung ermöglicht. Durch Regressionsberechnung kann aus dem Ergebnis der ersten Hauptanalyt- Analyse der Wasserprobe auf diese Weise die tatsächliche und genaue Konzentration des Analyts in der Wasserprobe bestimmt werden.

Selbstverständlich können auch mehr als zwei Analytabschnitte mit entsprechenden Hilfssubstanzen beziehungsweise Analyt-Standards vorgesehen sein, um die Genauigkeit der Kalibrierung insbesondere bei nicht-linearen Kennlinien zu erhöhen.

Das Testelement kann physisch beispielsweise die Größenordnung eines breiten Streichholzes aufweisen. Die Probenleitung kann daher einen entsprechend kleinen Querschnitt haben, der im Bereich von 0,01 mm² bis zu mehreren Quadratmillimetern liegen kann. Der Fotometerabschnitt, beziehungsweise die von ihm gebildete Messstrecke, sollte möglichst lang sein, beispielsweise mehrere Millimeter bis zu mehreren Zentimetern. Das Volumen der Wasserprobe in dem Fotometerabschnitt liegt also im Bereich von einem bis zu circa hundert Kubikmillimetern. Entsprechend gering ist die hierfür erforderliche Reagenz-Menge. Damit ist auch die durch das Reagenz begründete Gefährdung für Umwelt beziehungsweise Gesundheit entsprechend gering. Hierdurch kann die Notwendigkeit einer reglementierten Entsorgung entfallen und das Einmal-Testelement in im Hausmüll entsorgt werden, so dass ein erheblicher Aufwand für die reglementierte Entsorgung beziehungsweise die Rückführung des Testelementes zum Lieferanten beziehungsweise zum Hersteller entfällt.

Alle für die Qualität des Messergebnisses relevanten Arbeitsschritte der Wasseranalyse, beispielsweise die Dosierung des Analyts, die Vermischung des Analyts mit der Wasserprobe, das Abwarten der erforderlichen Reaktionszeit etc., erfolgen halbautomatisch beziehungsweise automatisch und verschlossen. Damit sind aus einer fehlerhaften Handhabung resultierende Fehler und Gefährdungen nahezu ausgeschlossen.

Vorzugsweise ist das Messgerät als Fotometer mit einer Lichtquelle zur Erzeugung eines Messstrahles und einem Lichtempfänger zum Empfangen des Lichtstrahles, nachdem dieser den einen Fotometerabschnitt bildenden Messabschnitt des Testelementes durchlaufen hat, ausgestattet. Besonders bevorzugt ist das Fotometer als Transmissions-Fotometer ausgebildet. Ein Transmissions-Photometer hat im Vergleich zum Reflektions-Fotometer ein relativ großes Nutzsignal. Dies ermöglicht auch bei einer relativ kurzen Messstrecke eine präzise quantitative Ermittlung des Analyts in der Wasserprobe. Der Messabschnitt weist mindestens ein Fotometer-Fenster für den Eintritt und den Austritt eines Fotometer-Messstrahles auf. Fotometrisch können beispielsweise Chlor, Phosphate und Ammonium bestimmt werden.

Alternativ kann das Messgerät ein elektrochemisches Messgerät sein, das eine elektrische Größe in dem Messabschnitt bestimmt. Der Messabschnitt weist mindestens eine Elektrode beziehungsweise Sensorfläche auf, die über Leiterbahnen mit Kontakten beziehungsweise Kontaktflächen des Testelementes verbunden ist, die wiederum mit entsprechenden Kontakten beziehungsweise Kontaktflächen der Basiseinheit, und auf diese Weise mit dem Messgerät der Basiseinheit verbunden sind. Die Elektrode beziehungsweise die Sensorfläche kann eine Fläche von wenigen Quadrat-Mikrometern bis zu einigen QuadratMillimetern aufweisen. Elektrochemisch können beispielsweise die Leitfähigkeit, das Redoxpotential, der pH-Wert und der Sauerstoffgehalt der Wasserprobe bestimmt werden.

Alternativ weist die Basiseinheit sowohl ein fotometrisches beziehungsweise optisches als auch ein elektrochemisches Messgerät auf, so dass sowohl ein optisches beziehungsweise fotometrisches als auch ein elektrochemisches Testelement in der Basiseinheit alternativ betrieben werden können.

Das Testelement kann mindestens ein Positionierelement aufweisen, das eine exakte Positionierung des Testelementes in der Basiseinheit gewährleistet. Um insbesondere eine fehlerfreie Fotometrie sicherstellen zu können, muss der Messabschnitt des Testelementes exakt mit dem Fotometer der Basiseinheit ausgerichtet sein. Hierzu ist mindestens ein separates Positionierelement vorgesehen, das neben den Seitenflächen des Testelementes für eine zusätzliche exakte Positionierung des Testelementes in der Basiseinheit sorgt. Beispielsweise kann in dem Testelement eine Nut, eine Vertiefung oder eine durchgehende Öffnung vorgesehen sein, in die ein entsprechendes vorgespanntes Rastelement der Basiseinheit eingreift, um das Testelement in der Basiseinheit exakt zu positionieren und zu fixieren.

Das Reagenz beziehungsweise die Reagenzien sind, beispielsweise in getrockneter Form, in der Probenleitung angeordnet. Selbstverständlich können auch mehrere verschiedene Hauptreagenzien, also Nachweisreagenzien, hintereinander in der Probenleitung hinter der Messstrecke vorgesehen sein. Sobald die Wasserprobe das betreffende Hauptreagenz erreicht, löst sich dieses in der Wasserprobe und reagiert mit dem zu bestimmenden Analyt beispielsweise farbverändernd.

Vorzugsweise weist das Einmal-Testelement eine Pumpenmembran auf, die von einem basiseinheitsseitigen Pumpenaktuator, der beispielsweise einen elektromotorisch angetriebenen Pumpenstößel aufweist, betätigt wird. Die flexible Pumpenmembran ist an dem zur Proben-Einlassöffnung gegenüberliegenden Ende der Probenleitung angeordnet, und schließt die Pumpöffnung der Probenleitung pneumatisch dicht ab, so dass durch Verformen der Pumpenmembran Luft verdrängt und die Wasserprobe in der Probenleitung bewegt werden kann. Durch Eindrücken beziehungsweise Deformation der Membran wird Flüssigkeit rückwärts und durch Entspannung der Membran, wird Flüssigkeit vorwärts in der Probenleitung bewegt. Die Entspannung der Membran kann entweder durch elastische Rückverformung der Membran oder durch Zurückziehen der Membran mittels des Pumpenaktuators geschehen. Die Pumpenmembrane und der Pumpenaktuator bilden also eine Membranpumpe, wobei lediglich die Pumpenmembrane an dem Einmal-Testelement vorgesehen ist. Der motorgetriebene Pumpenaktuator bzw. Pumpenstößel ist basiseinheitseitig angeordnet.

Alternativ ist testelementseitig lediglich eine Pumpöffnung der Probenleitung vorgesehen. Die Pumpöffnung ist an dem der Einlassöffnung gegenüberliegenden Ende der Probenleitung angeordnet. An der Basiseinheit ist eine Probenpumpe vorgesehen, die mit der Pumpöffnung des eingesteckten Testelementes verbunden ist, solange das Einmal-Testelement in die Basiseinheit eingesteckt ist.

Vorzugsweise ist ein Absorptionskörper zwischen dem Messabschnitt und der Pumpöffnung angeordnet. Der Absorptionskörper dient der Aufnahme der Wasserprobe, nachdem diese im Messabschnitt letztmalig analysiert und vorwärts zu dem Absorptionskörper gefördert wurde. Hierdurch wird die Wasserprobe immobilisiert, und ein Auslaufen der Wasserprobe aus dem Testelement heraus verhindert. Der Absorptionskörper kann beispielsweise ein Vlieskörper, ein Tonkörper beispielsweise aus Bentonit, oder ein so genannter Superabsorber sein. Der Absorptionskörper kann zusätzlich ein Neutralisationsreagenz enthalten.

Vorzugsweise ist in der Probenleitung ein Probenfilter angeordnet, der die durch die Einlassöffnung angesaugte Wasserprobe filtert, bevor diese zu dem Messabschnitt befördert und dort insbesondere fotometrisch analysiert wird. Der Probenfilter kann beispielsweise aus Glaswolle bestehen.

Vorzugsweise ist in der Probenleitung eine hydrophobe Stopperkapillare angeordnet. Die Stopperkapillare kann in der Nähe der Pumpöffnung angeordnet sein, so dass verhindert wird, dass die Wasserprobe die Probenleitung verlässt und durch die Pumpöffnung in die Basiseinheit gelangt. Anstatt der Stopperkapillare kann auch ein hydrophober Stopperfilter eingesetzt werden.

Gemäß einer bevorzugten Ausgestaltung ist in der Probenleitung zwischen der Einlassöffnung und dem Messabschnitt eine Dosierkapillare angeordnet. Bevorzugt ist die Dosierkapillare unmittelbar an der Einlassöffnung vorgesehen. Durch die Dosierkapillare wird bei der Wasserprobennahme die Wasserprobe im wesentlichen durch die Kapillarkraft der Dosierkapillare gefördert, und auf diese Weise eine definierte Wasserprobe-Menge isoliert. Erst anschließend hieran wird die Wasserprobe durch den Pumpenaktuator aktiv in der Probenleitung vorwärts beziehungsweise gegebenenfalls rückwärts gefördert.

An dem Testelement kann ein Trockenmittel angeordnet sein, um das Hauptreagenz vor Feuchtigkeit zu schützen. Das Trockenmittel kann beispielsweise durch eine hydrophobe Stopperkapillare von der Probenleitung getrennt sein, durch die hindurch Feuchtigkeit aus der Probenleitung zu dem Trockenmittel gelangen kann.

Die Einlassöffnung und/oder die Pumpöffnung können mit einer feuchtigkeitsdichten Transportversiegelung versiegelt sein, die beim Einstecken des Testelementes in die Basiseinheit automatisch und oder manuell geöffnet, beispielsweise durchgestochen wird. Alternativ oder ergänzend kann das Testelement einzeln in eine feuchtigkeitsdichte Verpackung eingeschweißt sein.

Im Folgenden werden unter Bezugnahme auf die Zeichnungen mehrere Ausführungsbeispiele der Erfindung näher erläutert.

Es zeigen:
- Figur 1: eine schematische Darstellung einer mobilen Wasser-Analyseanordnung, bestehend aus einer Basiseinheit und einem Testelement, zur Durchführung des erfindungsgemäßen Verfahrens
- Figur 2: das Testelement der Analyseanordnung der Figur 1,
- Figur 3: ein zweites Ausführungsbeispiel einer mobilen Wasser-Analyseanordnung einschließlich einer austauschbaren Kassette mit mehreren Testelementen, zur Durchführung des erfindungsgemäßen Verfahrens

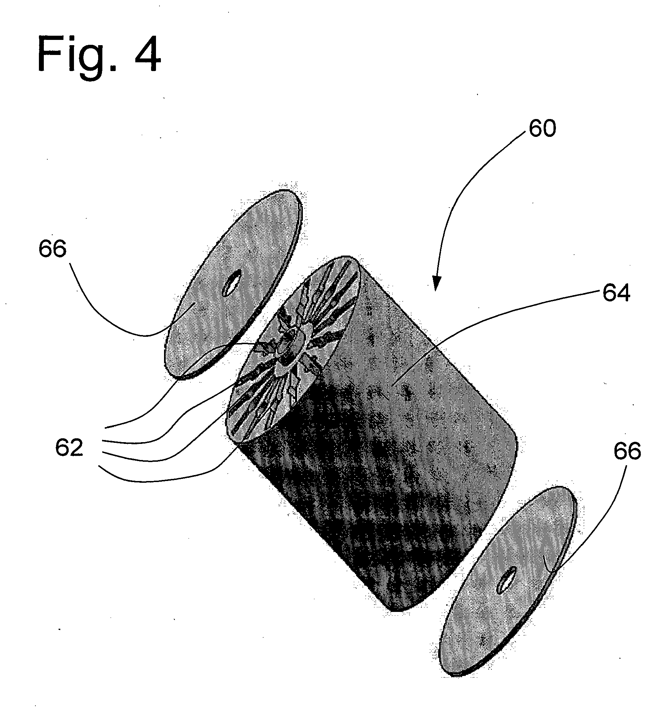

- Figur 4: die austauschbare Kassette der Figur 3,
- Figur 5: eine zweite Ausführungsform eines elektrochemischoptischen Testelementes in Vorderansicht,
- Figur 6: eine Rückansicht des Testelementes der Figur 5, und
- Figur 7: eine Seitenansicht einer dritten Ausführungsform eines Testelementes mit einer Pumpenmembrane, die von einem Pumpenaktuator der Basiseinheit betätigt wird.

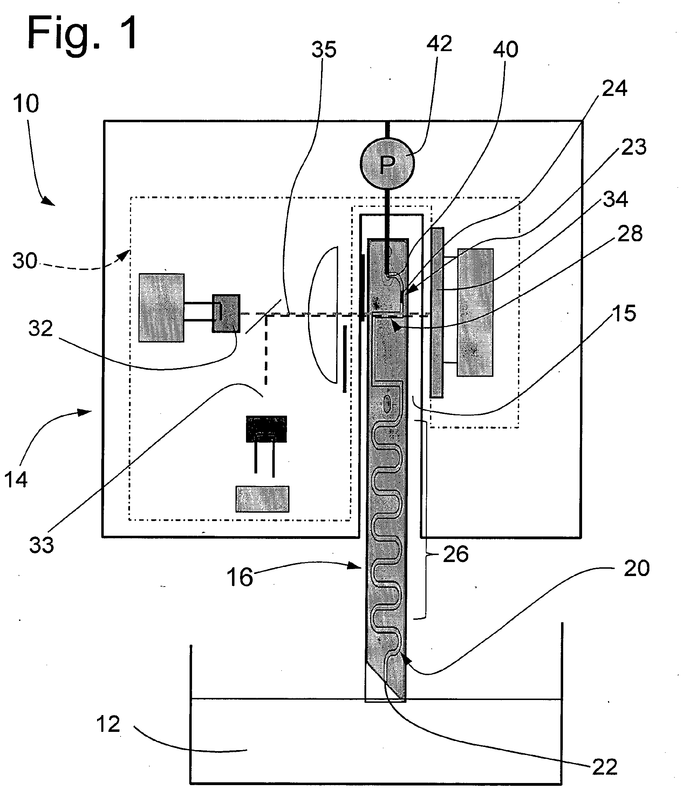

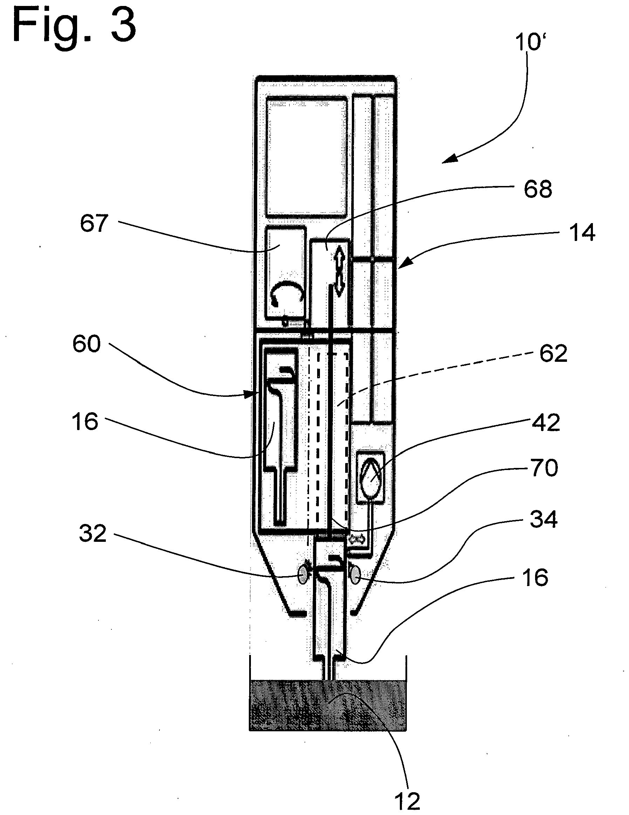

In den Figuren 1 und 3 ist jeweils eine mobile Wasser-Analyseanordnung 10,10' zur quantitativen Bestimmung eines Analyts in einer Wasserprobe schematisch dargestellt. Vorliegend ist eine fotometrische Analyseanordnung 10,10' dargestellt und beschrieben, mit der beispielsweise Chlor, Phosphat oder Ammonium bestimmt werden kann. Grundsätzlich kann die Analyseanordnung alternativ jedoch auch als elektrochemische Analyseanordnung mit einem elektrischen Messgerät ausgebildet sein.

Die Analyseanordnung der Figur 1 gliedert sich in eine Basiseinheit 14 und ein austauschbares Einmal-Testelement 16, das vorliegend in die Basiseinheit 14 eingesteckt ist.

Das Testelement 16 wird von einem Testelement-Körper 18 aus Kunststoff gebildet, in den eine Probenleitung 20 nutartig eingelassen ist. Der Testelement-Körper 18 ist auf der Seite der Nutöffnung durch eine nicht dargestellte Kunststoff- oder Aluminium- Folie verschlossen.

Die Probenleitung 20 weist an ihrem in Bezug auf die Basiseinheit 14 distalen Ende eine Einlassöffnung 22 auf, durch die eine Wasserprobe aus einem Wasservorrat 12 angesaugt werden kann. An die Einlassöffnung 22 schließt sich in Fließrichtung ein mäanderartig verlaufender Mischbereich 26 der Probenleitung 20 an, in dem ein Hauptreagenz, Hilfsreagenz oder eine Hilfssubstanz und die angesaugte Wasserprobe homogen miteinander vermischt werden können.

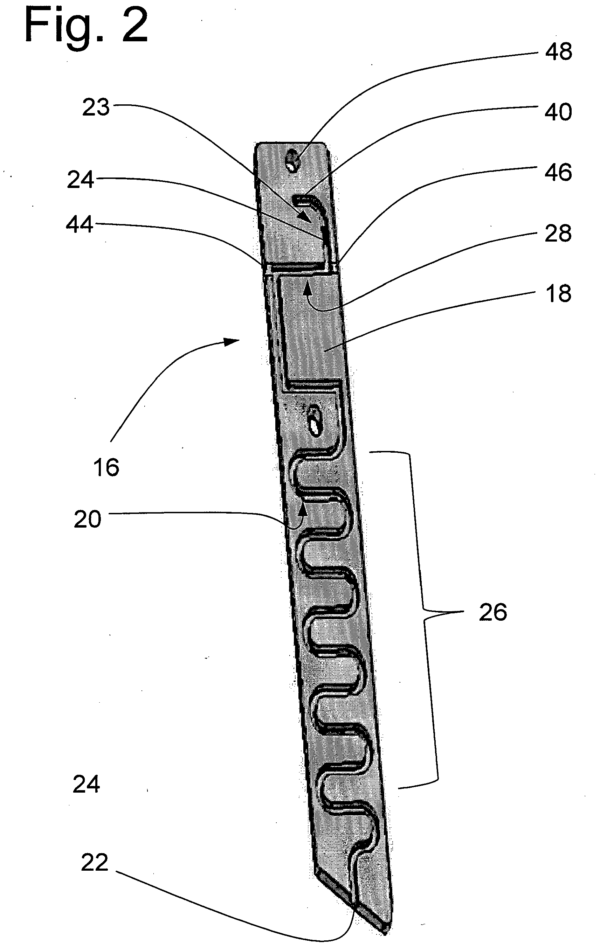

An den Mischbereich 26 schließt sich ein Messabschnitt 28 an, in dem eine quantitative Bestimmung vorgenommen werden kann. Der Messabschnitt 28 ist vorliegend ein Fotometerabschnitt, der die Messstrecke für ein entsprechendes Fotometer-Messgerät 30 der Basiseinheit 14 bildet. An beiden Enden des Fotometerabschnittes 28 ist jeweils ein für den Messstrahl transparentes Fenster 44,46 vorgesehen, wie in Figur 2 dargestellt. Der Testelement-Körper 18 kann vollständig aus einem für den Messstrahl 35 transparenten Kunststoff bestehen.

Proximal des Messabschnittes 28, also von der Einlassöffnung 22 aus gesehen hinter dem Messabschnitt 28, schließt sich ein erster Reagenzabschnitt 23 mit einem getrockneten Reagenz 24 an, das ein Hauptreagenz ist. An dem der Einlassöffnung 22 gegenüberliegenden Ende der Probenleitung 20, also hinter dem ersten Reagenzabschnitt 23, ist als Pumpelement eine Pumpöffnung 40 vorgesehen, die bei in die Basiseinheit 14 eingestecktem Testelement 16 mit einer einen Pumpenaktuator bildenden basiseinheitseitigen Probenpumpe 42 verbunden ist.

Die Basiseinheit 14 weist als Messgerät 30 ein Transmissions-Fotometer auf, das zwei Lichtquellen 32,33 verschiedener Wellenlänge sowie einen Lichtempfänger 34 für beide Wellenlängen aufweist.

Das Testelement 16 weist ein Positionierelement 48 auf, das als durchgehende Öffnung ausgebildet ist. Das Positionierelement 48 wirkt mit einem entsprechenden vorgespannten Rastelement der Basiseinheit 14 derart zusammen, dass das Testelement 16 exakt und reproduzierbar in der Basiseinheit 14 positioniert ist. Hierdurch wird sichergestellt, dass der von den Lichtquellen 32,33 generierte Messstrahl 35 exakt fluchtet mit dem fotometrischen Messabschnitt 28. Die Testelement-Aufnahme der Basiseinheit 14 wird von einem Aufnahme-Schacht 15 gebildet, in dem das Testelement 16 weitgehend spielfrei eingeschoben ist.

In der Figur 3 ist ein zweites Ausführungsbeispiel einer mobilen Wasser-Analyseanordnung 10' dargestellt, die eine austauschbare Kassette 60 aufweist, die als Trommel mit 15 Fächern 62 für jeweils ein Testelement 16 ausgebildet ist. Der Kunststoff-Trommelkörper 64 ist axial jeweils durch kreisförmige Versiegelungs- Folien 66 derart abgeschlossen, dass die Fächer 62 gas- und flüssigkeitsdicht versiegelt sind.

Wie in der Figur 3 dargestellt ist, ist die austauschbare Kassette 60 in eine entsprechende Kassetten-Aufnahme der Basiseinheit 14' eingesetzt. Die Basiseinheit 14' weist einen Kassetten-Drehantrieb 67 und einen Testelement-Schieberantrieb 68 mit einem Testelement-Schieber 70 auf. Der Schieber 70 kann automatisch ein Testelement 16 aus einer Kammer 62 in die in der Figur 3 dargestellte Messposition schieben.

Sobald die Messung beendet ist, schiebt der Schieber 70 das Testelement 16 aus der Messposition, und wirft dieses aus der Basiseinheit 14' aus. Anschließend wird der Schieber 70 vollständig aus der Kassette 60 herausgezogen, woraufhin der Drehantrieb 67 die Kassette 60 um einen Kammerwinkel weiterdreht, so dass die nächste ein Testelement 16 aufweisende Kammer 62 mit dem Schieber 70 fluchtet. Sobald der Benutzer einen Messwunsch signalisiert, schiebt der Schieber 70 das Testelement 16 aus der Klammer 62 in die Messposition, und die Messung kann beginnen.

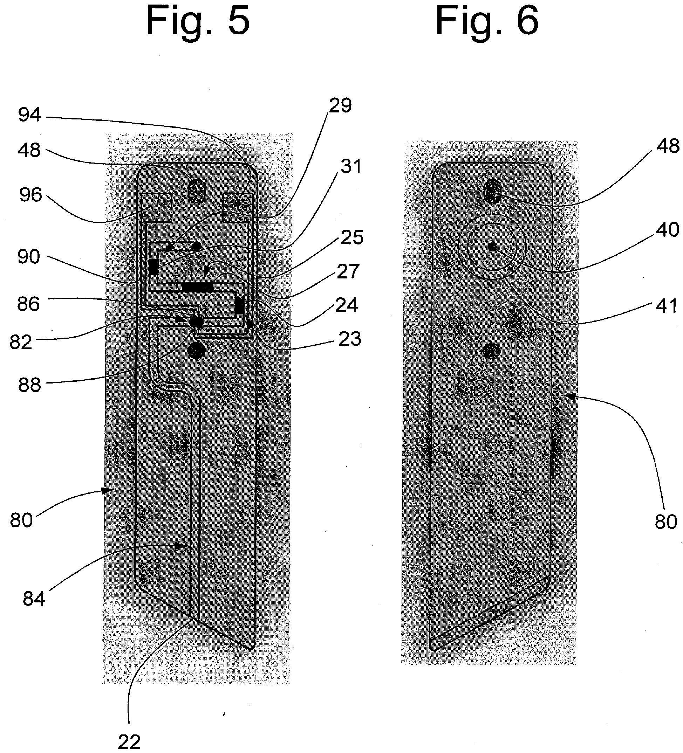

In den Figuren 5 und 6 sind die Vorderseite und die Rückseite einer zweiten Ausführungsform eines Testelementes 80 dargestellt. Das Testelement 80 ist ein elektrochemisch-optisches Testelement, das einen elektrochemisch-optischen Messabschnitt 82 im Verlauf der Probenleitung 84 aufweist. An dem Messabschnitt 82 sind zwei einander gegenüberliegende Elektroden 86,88 vorgesehen, die über Leiterbahnen 90,92 mit Kontakten 94,96 verbunden sind. Die Kontakte 94,96 liegen entsprechenden Kontakten der Basiseinheit gegenüber, wobei letztere mit dem elektrochemischen Messgerät der Basiseinheit verbunden sind. Ferner weist der Messabschnitt 82 eine optische Messtrecke für ein Fotometer auf.

Von der Einlassöffnung 22 aus gesehen hinter dem Messabschnitt 82 schließt sich ein erster Reagenzabschnitt 23 mit einem ersten Reagenz 24 an, das ein Hauptreagenz 24 ist. Hinter dem ersten Reagenzabschnitt 23 schließt sich ein zweiter Reagenzabschnitt 25 mit einem zweiten Reagenz 27 in Form einer ersten Hilfssubstanz 27 an. An den zweiten Reagenzabschnitt 25 schließt sich ein dritter Reagenzabschnitt 29 mit einem dritten Reagenz 31 in Form einer zweiten Hilfssubstanz 31 an. Die beiden Hilfssubstanzen 27,31 sind jeweils Analyt-Standards verschiedener Menge beziehungsweise verschiedener Konzentration.

Auf der in der Figur 6 dargestellten Testelement-Rückseite ist das als Pumpöffnung 40 ausgebildete Pumpelement des Testelementes 80 dargestellt. Zur Herstellung einer weitgehend gasdichten Anbindung der Probenleitung 84 mit dem als Pumpe ausgebildeten Pumpenaktuator 42 ist ein ringförmiges Dichtelement 41 vorgesehen, das die Pumpöffnung 40 umgibt.

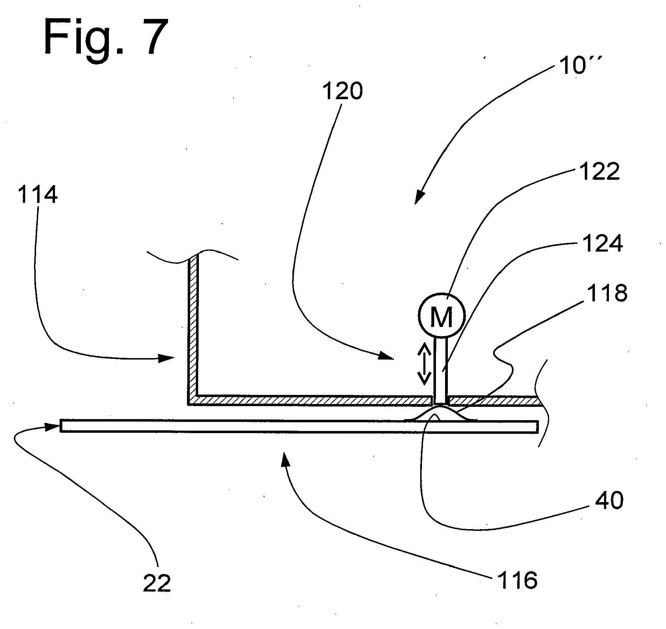

In der Figur 7 ist in Seitenansicht schematisch und nur teilweise ein weiteres Ausführungsbeispiel einer mobilen Wasser-Analyseanordnung 10" dargestellt. Die Wasser-Analyseanordnung 10" weist ein Einmal-Testelement 116 auf, das über der Pumpöffnung 40 eine konvexe blasenförmige Pumpenmembrane 118 aufweist. Die ein Pumpelement bildende Pumpenmembrane 118 schließt ein Volumen ein, das größer ist als das gesamte Volumen der Probenleitung 84.

Die Basiseinheit 114 weist als Pumpenaktuator 120 einen Antriebsmotor 122 und einen hiervon angetriebenen Pumpenstößel 124 auf, der die Pumpenmembrane 118 des eingesteckten Einmal-Testelements 116 fein dosierend betätigt. Durch entsprechende Deformation der Pumpenmembrane durch den Pumpenstößel 124 kann die Wasserprobe über die gesamte Länge der Probenleitung beliebig oft vorwärts und rückwärts gefördert werden.

Zur Bestimmung eines Analyts in einer Wasserprobe wird zunächst ein Testelement 16 in die Testelement-Aufnahme der Basiseinheit 14 eingesteckt. Hierdurch wird gegebenenfalls die Basiseinheit 14 eingeschaltet. Daraufhin wird die Einlassöffnung 22 des Testelementes 16 manuell in den zu untersuchenden Wasservorrat 12 eingetaucht, und durch die Pumpe 42 eine Wasserprobe durch Vorwärtsfördern in der Probenleitung 20 bis zu dem Messabschnitt 28 gefördert. In dem Messabschnitt 28 wird mit Hilfe des Messgerätes 30 in einer ersten Analyse der Probenblindwert der Wasserprobe bestimmt.

Sobald die Probenblindwert-Bestimmung abgeschlossen ist, wird die Wasserprobe aus dem Messabschnitt 28 vorwärts in den ersten Reagenzabschnitt 23 gefördert. In dem ersten Reagenzabschnitt 23 stößt die Wasserprobe auf das als Hauptreagenz ausgebildete erste Reagenz 24, und vermischt sich dort mit dem Hauptreagenz. Das Hauptreagenz geht mit dem nachzuweisenden Analyt in der Wasserprobe eine Reaktion ein, die die optischen Absorptions-Eigenschaften der Wasserprobe verändert.

Durch Rückwärtsfördern wird die Wasserprobe von dem ersten Reagenzabschnitt 23 wieder zurück in den Messabschnitt 28 gefördert. Dort wird die Wasserprobe durch das Messgerät 30 fotometrisch zum zweiten Mal analysiert. Das Ergebnis dieser zweiten Analyse ist ein Brutto-Messwert. Schließlich wird der Blindproben-Messwert von dem Brutto-Messwert subtrahiert, um auf diese Weise den Netto-Analyt-Messwert und damit die Konzentration des Analyts in der Wasserprobe zu erhalten.

Mit dem in den Figuren 5 und 6 dargestellten Testelement 80 kann darüber hinaus mit Hilfe des Standard-Additionsverfahrens die Bestimmung des Analyts in der Wasserprobe noch erheblich präzisiert werden. Hierzu wird die Wasserprobe nach der Bestimmung des Analyts in dem Messabschnitt 82 erneut vorwärts bis zu dem zweiten Reagenzabschnitt 23 gefördert, wo ein zweites Reagenz 27 angeordnet ist. Das zweite Reagenz 27 ist eine erste Hilfssubstanz, die einen Analyt-Standard bildet. Die Wasserprobe vermischt sich mit dem zweiten Reagenz 27 in dem zweiten Reagenzabschnitt 25. Anschließend wird die Wasserprobe aus dem zweiten Reagenzabschnitt 25 durch Rückwärtsfördern zurück zu dem Messabschnitt 82 gefördert, wo die Wasserprobe zum dritten Mal fotometrisch analysiert wird. Schließlich wird die Wasserprobe erneut vorwärts bis in den dritten Reagenzabschnitt 29 gefördert, wo sie sich mit dem dritten Reagenz 31, das als ein zweites Hilfsreagenz in Form eines zweiten Analyt-Standards ausgebildet ist, vermischt. Von dem dritten Reagenzabschnitt 29 wird die Wasserprobe wieder rückwärts zurück zu dem Messabschnitt 82 gefördert, wo sie zum viertem Mal fotometrisch analysiert wird.

Aus den beiden fotometrischen Analyt-Standard- Analysen kann eine Konzentrations-Absorptions- Kennlinie generiert werden, die eine genaue Bestimmung der Analyt-Konzentration der Wasserprobe aus dem Netto-Analyt- Messwert erlaubt.

Alternativ können die Reagenzien 27,31 auch Hilfsreagenzien anderer Art sein:
Das Hilfsreagenz kann ein Neutralisationsreagenz sein. Nach der Bestimmung des Analyts in dem Messabschnitt 28 wird die Wasserprobe zu dem Hilfsreagenz gepumpt, das mit dem Hauptreagenz derart reagiert, dass das Hauptreagenz neutralisiert wird, und beispielsweise im Hausmüll entsorgt werden kann.

Das Hilfsreagenz kann auch gelierend und/oder verfärbend in der Wasserprobe wirken, und nach der Durchführung der quantitativen Bestimmung des Analyts mit der Wasserprobe zusammengeführt werden. Durch die Verfärbung wird deutlich sichtbar gemacht, dass das Testelement bereits benutzt wurde. Dies kann gegebenenfalls auch durch das Basisgerät festgestellt werden. Durch die Gelierung wird die Wasserprobe in der Probenleitung 20 fixiert, und kann nicht mehr auslaufen.

Das Hilfsreagenz kann auch als Aktivator für das Hauptreagenz ausgebildet sein. Hierzu sind das Hauptreagenz und das Hilfsreagenz hintereinander angeordnet. Das Hilfsreagenz aktiviert das Hauptreagenz, sobald es mit dem Hilfsreagenz in der Wasserprobe vermischt wird. Sowohl das Hauptreagenz als auch das Hilfsreagenz können zwischen dem Messabschnitt 28 und der Pumpöffnung 40 angeordnet sein.

## Patentansprüche

1. Verfahren zur Bestimmung eines Analyts in einer Wasserprobe mit einer mobilen Wasser-Analyseanordnung (10) mit einer mobilen Basiseinheit (14) und einem austauschbaren Einmal-Testelement (16), das in die Basiseinheit (14) eingesteckt ist, wobei
das Einmal-Testelement (16) umfasst:
eine Probenleitung (20) mit einer Einlassöffnung (22) zur Aufnahme der Wasserprobe, einem die Messstrecke bildenden Messabschnitt (28) zur Bestimmung eines Analyts der Wasserprobe und einem von der Einlassöffnung (22) aus gesehen fluidisch hinter dem Messabschnitt (28) liegenden ersten Reagenzabschnitt (23), und
ein Reagenz (24), das in der Probenleitung (20) in dem ersten Reagenzabschnitt (23) angeordnet ist,
und die Basiseinheit (14) umfasst:
eine Testelement-Aufnahme zum Halten des eingesteckten Testelementes (16),
ein Messgerät (30), dessen Messstrecke von dem Testelement-Messabschnitt (28) gebildet wird, und
eine Pumpe (42) oder einen Pumpenaktuator (120) zum Fördern der Wasserprobe,
mit den Verfahrensschritten:
Einstecken des Testelementes (16) in die Testelement-Aufnahme der Basiseinheit (14),
Vorwärtsfördern der Wasserprobe von der Einlassöffnung (22) bis zu dem Messabschnitt (28), wodurch die Wasserprobe reagenzfrei in den Messabschnitt (28) gelangt,
erstes Analysieren der reagenzfreien Wasserprobe in dem Messabschnitt (28) mit Hilfe des Messgerätes (30) und Ermittlung eines Probenblindwertes,
Vorwärtsfördern der Wasserprobe aus dem Messabschnitt (28) in den ersten Reagenzabschnitt (23),
Zurückfördern der Wasserprobe aus dem ersten Reagenzabschnitt (23) in den Messabschnitt (28),
zweites Analysieren der Wasserprobe in dem Messabschnitt (28) mit Hilfe des Messgerätes (30), und
Subtrahieren des Probenblindwerts der ersten Analyse von dem Messwert der zweiten Analyse, um die Konzentration des Analyts in der Wasserprobe zu erhalten.

2. Verfahren nach Patentanspruch 1, wobei in der Probenleitung (84) jenseits des ersten Reagenzabschnittes (23) ein zweiter Reagenzabschnitt (25) mit einem zweiten Reagenz vorgesehen ist, das bevorzugt eine Hilfssubstanz (27) ist, mit dem sich an die Wasserproben-Analyse anschließenden Schritt:
Vorwärtsfördern der analysierten Wasserprobe in den zweiten Reagenzabschnitt (25).

3. Verfahren nach Patentanspruch 2, mit dem sich an das Vorwärtsfördern der analysierten Wasserprobe in den zweiten Reagenzabschnitt (25) anschließenden Verfahrensschritt:
Rückwärtsfördern der Wasserprobe aus dem zweiten Reagenzabschnitt (25) in den Messabschnitt (82), und
drittes Analysieren der Wasserprobe mit Hilfe des Messgerätes (30).

4. Verfahren nach Patentanspruch 3, mit einem dritten Reagenzabschnitt (29) mit einem dritten Reagenz (31) hinter dem zweiten Reagenzabschnitt (25), wobei das dritte Reagenz bevorzugt eine Hilfssubstanz (31) ist, wobei sich folgende Verfahrensschritte an das dritte Analysieren der Wasserprobe anschließen:
Vorwärtsfördern der Wasserprobe in den dritten Reagenzabschnitt (29),
Rückwärtsfördern der Wasserprobe aus dem dritten Reagenzabschnitt (29) in den Messabschnitt (82), und
viertes Analysieren der Wasserprobe in dem Messabschnitt (82) mit Hilfe des Messgerätes (30).

## Claims

1. A method for determining an analyte in a water sample with a mobile water analyzing system (10) having a mobile basic unit (14) and an exchangeable single-use test element (16) inserted in the basic unit (14), wherein the single-use test element (14) comprises:
a sample line (20) with an inlet opening (22) configured to receive the water sample, a measuring section (28) forming a measuring track and configured to allow determination of an analyte in the water sample, and a first reagent section (23) fluidically disposed behind the measuring section (28), seen from the inlet opening (22), and
a reagent (24) disposed in the first reagent section (23) of the sample line (20);
and the basic unit (14) comprises:
a test element receptacle configured to hold the inserted test element (16), and
a measuring device (30) with a measuring track formed by the measuring section (28) of the test-element, and
a pump (42) or a pump actuator (120) for conveying the water sample,
comprising the following method steps:
inserting the test element (16) into the test element receptacle of the basic unit (14);
transporting the water sample forward from the inlet opening (22) to the measuring section (28), whereby the water sample reaches the measuring section (28) free of reagent,
performing a first analysis of the reagent-free water sample in the measuring section (28) using the measuring device (30) and determining a blank value of the sample,
transporting the water sample forward from the measuring section (28) into the first reagent section (22),
transporting the water sample backward from the first reagent section (22) into the measuring section (28),
performing a second analysis of the water sample in the measuring section (28) using the measuring device (30), and
subtracting the sample blank value of the first analysis from the measured value of the second analysis to obtain the concentration of the analyte in the water sample.

2. The method as recited in claim 1, wherein the sample line (84) further comprises, disposed beyond the first reagent section (23), a second reagent section (25) with a second reagent, which preferably is an auxiliary substance (27), the method further comprising, after performing the analysis of the water sample:
transporting an analyzed water sample forward into the second reagent section (25).

3. The method as recited in claim 2, wherein, after transporting the analyzed water sample forward into the second reagent section (25), the method further comprises:
transporting the water-sample backward from the second reagent section (25) into the measuring section (82), and
performing a third analysis of the water sample using the measuring device (30).

4. The method as recited in claim 3, wherein a third reagent section (29) with a third reagent (31) is disposed behind the second reagent section (25), the third reagent preferably being an auxiliary substance (31), wherein, after performing the third analysis of the water sample, the method further comprises the following steps:
transporting the water sample forward into the third reagent section (29),
transporting the water sample backward from the third reagent section (29) into the measuring section (82), and
performing a fourth analysis of the water sample in the measuring section (82) using the measuring device (30).

## Revendications

1. Procédé d'analyse d'un analyte dans un échantillon d'eau à l'aide d'un dispositif d'analyse d'eau (10) mobile comportant une unité de base (14) mobile et un élément de test (16) jetable inséré dans ladite unité de base (14), ledit élément de test (16) jetable comportant:
une conduite à échantillon (20) avec une ouverture d'admission (22) pour recevoir ledit échantillon d'eau, une zone de mesure (28), formant la section de mesure, pour déterminer un analyte dans l'échantillon d'eau et une première zone de réactif (23) située fluidiquement en aval de la zone de mesure (28), vu de l'ouverture d'admission (22), et
un réactif (24) disposé dans ladite première zone de réactif (23) dans la conduite à échantillon (20),
et ladite unité de base (14) comportant:
un logement pour l'élément de test pour retenir ledit élément de test (16) inséré,
un appareil de mesure (30) dont la section de mesure est formée par la zone de mesure (28) de l'élément de test, et
une pompe (42) ou un actionneur de pompe (120) pour refouler l'échantillon d'eau,
le procédé comportant les étapes suivantes:
l'insertion de l'élément de test (16) dans ledit logement pour l'élément de test de ladite unité de base (14),
le refoulement en avant dudit échantillon d'eau de l'ouverture d'admission (22) jusqu'à la zone de mesure (28), ledit échantillon d'eau arrivant dans la zone de mesure (28) exempte de réactif,
une première analyse de l'échantillon d'eau exempte de réactif dans la zone de mesure (28) à l'aide de l'appareil de mesure (30), et la détermination d'une valeur à blanc dudit échantillon,
le refoulement en avant dudit échantillon d'eau de la zone de mesure (28) dans la première zone de réactif (23),
le refoulement inverse dudit échantillon d'eau de la première zone de réactif (23) dans la zone de mesure (28),
une deuxième analyse de l'échantillon d'eau dans la zone de mesure (28) à l'aide de l'appareil de mesure (30), et
la soustraction de la valeur à blanc dudit échantillon de la première analyse de la valeur de la deuxième analyse pour obtenir la concentration dudit analyte dans l'échantillon d'eau.

2. Procédé selon la revendication 1, dans lequel une deuxième zone de réactif (25) avec un deuxième réactif est disposée au-delà de la première zone de réactif (23), le deuxième réactif étant, de préférence, une substance auxiliaire (27), le procédé comportant l'étape suivante après l'analyse de l'échantillon d'eau:
le refoulement en avant dudit échantillon d'eau analysé dans la deuxième zone de réactif (25).

3. Procédé selon la revendication 2, avec l'étape suivante après le refoulement en avant dudit échantillon d'eau analysé dans la deuxième zone de réactif (25):
le refoulement inverse dudit échantillon d'eau de la deuxième zone de réactif (25) dans la zone de mesure (82), et
une troisième analyse de l'échantillon d'eau à l'aide de l'appareil de mesure (30).

4. Procédé selon la revendication 3, avec une troisième zone de réactif (29) ayant un troisième réactif (31), disposée en aval de la deuxième zone de réactif (25), le troisième réactif étant, de préférence, uns substance auxiliaire (31), le procédé comportant l'étape suivante après la troisième analyse de l'échantillon d'eau:
le refoulement en avant dudit échantillon d'eau analysé dans la troisième zone de réactif (29),
le refoulement inverse dudit échantillon d'eau de la troisième zone de réactif (29) dans la zone de mesure (82), et
une quatrième analyse de l'échantillon d'eau dans la zone de mesure (82) à l'aide de l'appareil de mesure (30).
